(19) Europäisches Patentamt / European Patent Office / Office européen des brevets

(11) **EP 2 611 414 B1**

(12) **EUROPEAN PATENT SPECIFICATION**

(45) Date of publication and mention
of the grant of the patent:
**12.11.2014 Bulletin 2014/46**

(51) Int Cl.:
**A61K 9/00** *(2006.01)*  **A61K 9/107** *(2006.01)*
**A61K 47/14** *(2006.01)*

(21) Application number: **11749460.9**

(22) Date of filing: **02.09.2011**

(86) International application number:
**PCT/EP2011/065236**

(87) International publication number:
**WO 2012/028733 (08.03.2012 Gazette 2012/10)**

(54) **A WATER-IN-OIL TYPE EMULSION FOR TREATING A DISEASE OF THE EYE**

WASSER-IN-ÖL-EMULSION ZUR BEHANDLUNG VON AUGENERKRANKUNGEN

ÉMULSION DE TYPE EAU-DANS-L'HUILE DESTINÉE AU TRAITEMENT D'UNE MALADIE OCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2010 US 875805**
**03.09.2010 EP 10175337**

(43) Date of publication of application:
**10.07.2013 Bulletin 2013/28**

(73) Proprietor: **Santen SAS**
**91000 Evry (FR)**

(72) Inventors:
• **LALLEMAND, Frédéric**
**F-94260 Fresnes (FR)**
• **GARRIGUE, Jean-Sébastien**
**F-91370 Verrières-le-Buisson (FR)**
• **PHILIPS, Betty**
**F-92160 Antony (FR)**

(74) Representative: **Icosa**
**83 avenue Denfert-Rochereau**
**75014 Paris (FR)**

(56) References cited:
**EP-A1- 1 867 323**   **WO-A2-01/93911**
**WO-A2-2009/039262**   **US-A1- 2006 121 115**

• **TAMILVANAN S: "Oil-in-water lipid emulsions: implications for parenteral and ocular delivering systems", PROGRESS IN LIPID RESEARCH, PERGAMON PRESS, PARIS, FR, vol. 43, no. 6, 1 November 2004 (2004-11-01), pages 489-533, XP004621515, ISSN: 0163-7827, DOI: DOI: 10.1016/J.PLIPRES.2004.09.001**
• **CHAN ET AL: "Phase transition water-in-oil microemulsions as ocular drug delivery systems: In vitro and in vivo evaluation", INTERNATIONAL JOURNAL OF PHARMACEUTICS, ELSEVIER BV, NL, vol. 328, no. 1, 1 December 2006 (2006-12-01), pages 65-71, XP005787883, ISSN: 0378-5173, DOI: DOI:10.1016/J.IJPHARM.2006.10.004 cited in the application**

## Description

### FIELD OF INVENTION

The present invention relates to the field of the treatment of the conditions or diseases of the eye through the intraocular administration of therapeutic agents.

### BACKGROUND OF INVENTION

The treatment of eye diseases by injecting a therapeutic agent directly in the vitreous chamber has shown promising results in the past. Macugen® (oligonucleotide) and Lucentis® (monoclonal antibody) are pharmaceutical products which are efficient to treat retinal diseases.

However, their half-life in the vitreous is relatively short, leading to repeated injections to maintain the effect. The rapid clearance of these products is due to the renewal of the vitreous liquid over time.

This issue was already addressed in the prior art: for example, WO2009/046198 describes a method for administering a therapeutic agent in the vitreous with a sustained release kinetic; this method involves the formation of a macroscopic gel-like structure comprising said therapeutic agent, in the vitreous chamber. Also, EP2187980 describes the injection in the vitreous chamber of a therapeutic agent combined with a polymeric precursor, which will form *in situ* a hydrogel suitable for controlled release of said therapeutic agent.

However, the injection in the vitreous of a subject of a gel or gel-like structure as described in these patent applications may cause visual discomfort to the subject due to the invasion of the visual field by said gel or gel-like structure.

In further prior art documents, a solid implant may be injected in the eye of the subject for release of the active ingredient over several months. However, this form of administration introduces a solid body within the eye of the patient, which in some cases is not wished. Moreover, this approach is more adapted for administration of lipophilic agents than for administration of hydrophilic agents, and may not be selected for administration of biological agents such as proteins and monoclonal antibodies.

Therefore, there remains a need for a method for sustaineously releasing in the vitreous chamber, a composition comprising a hydrophilic therapeutic agent, such as for example a protein or a nucleic acid. Ensuring the visual comfort of the patient when the composition is within the vitreous body is another issue.

Surprisingly, the Applicant realized that a water-in-oil emulsion could be an efficient vehicle for administering hydrophilic therapeutic agents. Water-in-oil type emulsions are biphasic systems in which water droplets are dispersed within an oil phase.

The use of water-in-oil type emulsions as vehicles for sustained release of therapeutic agents is well known in the art. For example, WO01/89479 discloses the use of water-in-oil type emulsions for the parenteral administration of hydrophilic active ingredients with a sustained release kinetic.

Tamilvanan (Progress in Lipid Research 43 (2004) 489-533) describes in general terms lipid emulsions as drug delivery vehicles for administration by intraocular injection.

This invention thus relates to the use of water-in-oil type emulsions for intraocular administration of a therapeutic agent to a subject in need thereof, providing a sustained release kinetic, and avoiding any invasion of the field of vision of the subject in his/her everyday life or safety issues.

An advantage of the solution proposed by the Applicant is that when injected intraocularly, the water-in-oil emulsion of the invention forms a reservoir of therapeutic agent that may be in the form of a layer or in the form of a bubble, having a lower density than the vitreous liquid. Therefore, when injected, the composition will rapidly (within 0.5 seconds to 1 minute) shift up from injection location to the upper part of the vitreous. Consequently, this liquid reservoir will float over the vitreous, out of the visual field, avoiding any visual discomfort for the subject to which the composition is administered. The therapeutic agent is then sustaineously released from the reservoir over a period of time ranging from two weeks to 6 months. The composition of the invention has the further advantage of being in physical contact with both vitreous body and targeted tissues such as, for example, the choroid or the retina, resulting in a targeted release of the therapeutic agent.

### DEFINITIONS

In the present invention, the following terms have the following meanings:

- **"Emulsion"**: colloidal system made of two non-miscible elements, for example oil and water. One element (the dispersed phase) is present on the form of droplets dispersed in the other element, constituting the continuous phase.

- **"Water-in-oil type emulsion"**: emulsion made of water or aqueous droplets (i.e. the dispersed phase) dispersed in an oil phase (i.e. the continuous phase). A water-in-oil type emulsion also comprises surfactants (as defined hereafter), to avoid phase separation.

- **"Sustained release kinetic":** describes the slow release kinetic of a compound, at a predetermined rate and over an extended period of time.

- **"Intraocular administration":** injection of a product directly in the eyeball i.e. injection in the anterior chamber or in the posterior cavity (vitreous cavity) of the eye.

- **"Surfactant":** defines a substance that lowers the interfacial tension between two liquids.

- **"Bioresorbable":** defines a compound that progressively disappears in a biological environment.

- **"Therapeutic agent":** describes a molecule or a substance, preferably a biological molecule such as for example an oligonucleotide, a siRNA, a miRNA, a DNA fragment, an aptamer, a peptide, an antibody, a protein and the like, or a chemical entity, having the capacity, when administered in a suitable amount, of slowing down or stopping the progression, aggravation, or deterioration of one or more symptoms of a disease or condition; alleviates the symptoms of a disease or condition; cures a disease or condition.

- **"Therapeutically effective amount":** the amount of a therapeutic agent necessary and sufficient for slowing down or stopping the progression, aggravation, or deterioration of one or more symptoms of the disease or condition; alleviating the symptoms of the disease or condition ; curing the disease or condition.

- **"Hydrophilic":** defines a molecule or a portion of a molecule that is typically charge-polarized and capable of hydrogen bonding, enabling it to dissolve more readily in water than in oil or other solvents.

- **"Lipophilic":** refers to a chemical compound capable to dissolve in fats, oils, lipids, and non-polar solvents.

- **"Non-miscible":** liquid which does not combine or blend with another liquid, or which does not combine or blend immediately with another liquid.

- **"implant"** is a solid dosage form which is implanted in a biological tissue usually composed of a polymer in which an active ingredient in incorporated to be slowly release.

- **"reservoir"** is a reserve of active ingredient which can be solid of liquid.

## SUMMARY

This invention relates to a composition for use in the treatment of a patient in need thereof by intraocular route by intraocular injection, of diseases or conditions of the eye, wherein the composition is a water-in-oil type emulsion comprising an oil phase, a lipophilic surfactant dissolved in the oil phase, an aqueous phase dispersed in the oil phase, a hydrophilic therapeutic agent dissolved in the aqueous dispersed phase, wherein the composition has a density lower than 1, preferably ranging from 0.91 to 0.97 g/cm$^3$. The density was measured by filling a calibrated volumetric flask with the emulsion and weighed on a balance. Volume/mass ratio is then calculated.

The viscosity of the composition ranges from 25 to 10 000 mPa.s at 20 °C, as measured using a Kinexus Pro from Malvern U.K. at 20°C.

The mean water droplet size ranges from 20 nm to 600 nm. In an embodiment, the mean water droplet size ranges from 25 nm to 500, preferably 30 to 200, more preferably 50-100 nm. In another embodiment, the mean water droplet size ranges from 20 nm to 100 nm. The mean particle size of the emulsions droplets was determined by quasi-elastic light scattering after dilution in water using a High Performance Particle Sizer (Malvern Instruments, UK).

The composition sustaineously releases the hydrophilic therapeutic agent within the eye.

The composition of the invention is for use in the treatment, by intraocular route, of diseases or conditions of the eye of a patient, wherein the composition is a water-in-oil type emulsion comprising an oil phase, a lipophilic surfactant dissolved in the oil phase, an aqueous phase dispersed in the oil phase, a hydrophilic therapeutic agent dissolved in the aqueous dispersed phase, and wherein the composition has a density lower than 1, preferably ranging from 0.91 to 0.97 g/cm$^3$, and wherein the composition has a viscosity ranging from 25 to 10 000 mPa.s at 20 °C, and wherein the size of the droplets of water ranges from 20 nm to 600 nm, and wherein the use by intraocular route is intraocular injection.

According to an embodiment, the oil phase is selected from the group comprising triglycerides such as, for example, medium chain or long chain triglycerides, monoglycerides, diglycerides, vegetable oils or mineral oils.

Preferably, the lipophilic surfactant is selected from the group comprising sorbitan ester such as, for example, sorbitan stearate, sorbitan laurate and sorbitan monopalmitate; bentonite; glycerol monostearate; propylene glycol monolaurate and mixtures thereof. In a preferred embodiment, the aqueous phase is present in the composition of the invention in an amount ranging from 0.1 to 70% in weight to the total weight of the composition, preferably from 2 to 50% w/w, more preferably from 10 to 30% w/w. Preferably, the hydrophilic therapeutic agent is selected from the group comprising monoclonal antibodies (full or Fab fragment), such as for example ranibizumab, bevacizumab, trastuzumab, cituximab or rituximab;

anti-angiogenic molecules, such as for example pegaptanib;

ROCK (Rho-kinases) inhibitors, such as for example fasudil;

proteins such as anti-CD160 S-HLA-G or WNT3A protein which activates WNT (Wingless - Integration site) for survival of photoreceptor cells;

growth factors such as epithelium growth factors (EGF), anti-EGF or TGF (Transforming growth factor);

siRNA such as siRNA anti-arginase;

miRNA;

oligonucleotides such as antisens DNA or antisens RNA;

iron chelating molecules such as deferiprone and salicylaldehyde isonicotinoyl hydrazone;

anti-inflammatory molecules such as epigallocatechin gallate;

antibiotics for back of the eye infection such as linezolide, clavulamic acid, macrolides; anti-inflammatory molecules preferably selected from the group comprising cortico-steroids such as dexamethasone and its hydrophilic derivatives; and mixtures thereof.

In one embodiment of the invention, the composition further comprises a lipophilic therapeutic agent in the oil phase, said lipophilic therapeutic agent being selected from the group comprising cyclosporine A, lutein, alpha-tocopherol and dexamethasone palmitate.

According to the invention, the composition may further comprise viscosity modifying agents, such as, for example an hydrogel of sodium hyaluronate, carbopol gels, hydroxyethyl cellulose, dextran, carboxymethyl cellulose, polyethylene glycol, polyvinyl alcohol, collagen, and/or pH buffering agents, such as, for example, phosphate, citrate, tris, histidine or acetate buffer, and/or osmolality modifying agents, such as, for example NaCl, KCl, $CaCl_2$, glycerol, mannitol, alpha-trehalose or propylene glycol.

In a preferred embodiment, the composition of the invention is intravitreally injectable.

The diseases or conditions of the eye that may be treated with the composition of the invention are preferably selected from the group comprising glaucoma, anterior uveitis, retinal oxidation, age related macular degeneration, posterior uveitis, diabetic macular edema and central vein occlusion.

This invention also relates to a pharmaceutical composition comprising the composition of the invention, and further comprising one or more pharmaceutically acceptable excipients.

This invention also relates to a medicament comprising a water-in-oil type emulsion as described above.

This invention also relates to a composition for use in the treatment by intraocular route of diseases or conditions of the eye according to claims 1-9

which comprises administering to a patient in need thereof by intraocular route, a composition of the invention, wherein a therapeutic amount of a hydrophilic therapeutic agent is dissolved in the aqueous dispersed phase. In the invention, said therapeutic agent is sustaineously released within the eye of the patient.

In an embodiment, the volume of the injected composition ranges from 5 to 250 microliters.

In an embodiment, the composition or the medicament is injected in the vitreous chamber or in the anterior chamber of the eye of the patient.

This invention also relates to a device comprising the composition or the medicament according to the invention. According to an embodiment, the device comprises a volume of 20 to 350 microliters of the composition of the invention.

According to a preferred embodiment, the composition, the pharmaceutical composition, the medicament or the device are not implants.

## DETAILED DESCRIPTION

The invention thus relates to a composition for use in the treatment by intraocular route of diseases or conditions of the eye, wherein the composition is a water-in-oil type emulsion comprising an oil phase, a lipophilic surfactant dissolved in the oil phase, an aqueous phase dispersed in the oil phase and a hydrophilic therapeutic agent dissolved in the aqueous dispersed phase, the composition having a density lower than 1, a viscosity ranging from 25 to 10 000 mPa.s at 20 °C, wherein the mean size of the droplets of water ranges from 20 nm to 600 nm, said composition sustaineously releasing the hydrophilic therapeutic agent, and wherein the use by intraocular route is intraocular injection.

In one embodiment of the invention, the oil phase of the water-in-oil type emulsion comprises an oil selected from the group comprising triglycerides such as, for example semi-synthetic oils: medium chain triglycerides (MCT) or long chain triglycerides; monoglycerides, diglycerides or vegetable oils such as, for example, castor oil or mineral oils. According to a particular embodiment of the invention, the emulsion is deprived of ethyl oleate, soybean oil or mixture thereof.

In a particular embodiment of the invention, the amount of oil phase in the water-in-oil type emulsion ranges from 30 to 99.9% in weight to the weight of the total emulsion, preferably from 50 to 98% w/w, more preferably from 70 to 90% w/w.

In one embodiment of the invention, the emulsion comprises one or more lipophilic surfactants, in an amount sufficient for ensuring the water-in-oil type of the emulsion. In a particular embodiment of the invention, said lipophilic surfactants are selected from the group comprising sorbitan ester such as, for example, sorbitan stearate and sorbitan monopalmitate, bentonite, glycerin monostearate, glyceryl monooleate and propylene glycol monolaurate or mixtures thereof, poloxamer 188, poloxamer 282, poloxamer 407, tyloxapol, vitamin E D-polyethylene glycol succinate, cetostearyl alcohol, cholesterol, ethylene glycol palmitostearate, lauric acid, myristic acid, myristyl alcohol, linoleic acid, oleic acid, palmitic acid, stearic acid oleyl alcohol. According to one embodiment, the emulsion is deprived of at least one surfactant selected from the group consisting of sorbitan mono laurate, polyoxyethylene sorbitan mono oleate, polysorbate 20 (Tween 20), sorbitan trioleate (Span 85), phospholipids such as egg lecithin or mixture thereof.

In a particular embodiment of the invention, the HLB (hydrophilic - lipophilic Balance) of the surfactants of the composition ranges from 0 to 9, preferably from 2 to 8.

In a particular embodiment of the invention, the amount of lipophilic surfactants in the water-in-oil type emulsion ranges from 0.1 to 10% in weight to the weight of the total emulsion, preferably from 0.5 to 5% w/w, more preferably from 1 to 2% w/w.

In one embodiment of the invention, the aqueous phase in the water-in-oil type emulsion is present in an amount ranging from 0.1 to less than 50% in weight to the weight of the total emulsion, preferably from 0.5 to 15% w/w, more preferably from 2 to 10% w/w. Preferably, said aqueous phase is water or is essentially composed of water.

In a particular embodiment of the invention, the composition includes one or more hydrophilic therapeutic agent(s) present in the aqueous droplets of the water-in-oil type emulsion.

In one embodiment of the invention, the hydrophilic therapeutic agent is selected from the group comprising monoclonal antibodies (full or fragment Fab), such as for example ranibizumab, bevacizumab trastuzumab, cituximab and rituximab; anti-angiogenic molecules, such as for example pegaptanib; a ROCK (Rho-kinases) inhibitor, such as for example fasudil; proteins such as anti-CD160 S-HLA-G; WNT3A protein which activates WNT (Wingless - Integration site) for survival of photoreceptor cells; growth factors such as epithelium growth factors (EGF), anti-EGF or TGF (Transforming growth factor); siRNA such as siRNA anti-arginase, miRNA; oligonucleotides such as antisens DNA or antisens RNA; iron chelating molecules such as deferiprone and salicylaldehyde isonicotinoyl hydrazone; anti-inflammatory molecules such as epigallocatechin gallate; or antibiotics for back of the eye infection such as linezolide, clavulamic acid, macrolides, anti-inflammatory molecules preferably selected from the group comprising cortico-steroids such as dexamethasone and its hydrophilic derivatives and mixtures thereof.

In an embodiment of the invention, the amount of hydrophilic therapeutic ingredient in the emulsion ranges from 0.01 to 10% in weight to the total weight of the emulsion, preferably from 0.05 to 5% w/w, more preferably from 0.1 to 1% w/w.

In a particular embodiment of the present invention, the hydrophilic therapeutic agent is not a drug complex comprising a therapeutic agent and a polymer.

In an embodiment of the invention, the emulsion further comprises one or more lipophilic therapeutic agents in the oil phase. In a preferred embodiment of the invention, said lipophilic therapeutic agent is selected from the group comprising cyclosporine A, lutein, alpha-tocopherol and dexamethasone palmitate.

In a preferred embodiment, the amount of lipophilic therapeutic ingredient in the emulsion ranges from 0.01 to 10% in weight to the total weight of the emulsion, preferably from 0.05 to 5% w/w, more preferably from 1 to 2% w/w.

In a particular embodiment of the present invention, the lipophilic therapeutic agent is not a drug complex comprising a therapeutic agent and a polymer.

In a particular embodiment of the present invention, the water-in-oil emulsion is deprived of at least one metabolic degradation enzyme inhibitors selected from the group consisting of CYP3A inhibitors, protease inhibitors like aprotinin, chymostatin, bacitracin, benzamidine, phosphoramidon, leupeptin, bestatin, cystatin, amastatin, pepstatin, potato carboxypeptidase, soybean trypsin inhibitor, diisopropylfluorophosphate or EDTA. In another particular embodiment of the present invention, the water-in-oil emulsion is deprived of at least one drug-efflux P-glycoprotein enzyme inhibitors selected from the group consisting of flavonoids contained in fruit juices such as naringenin, isoquercetin, quercetin or vitamin E tocopheryl glycolsuccinate (TPGS).

The water-in-oil type emulsion of the invention presents a lower density than the vitreous liquid which has a density equivalent, if not equal, to the density of water. According to the invention, the density of the water-in-oil type emulsion of the invention is less than 1. Preferably, the density of the water-in-oil type emulsion ranges from 0.90 to 0.99, preferably from 0.91 to 0.97, more preferably from 0.93 to 0.96. Therefore, when injected in the vitreous body, the emulsion will be located over the vitreous liquid.

Also, when injected, the composition will form a non-breakable reservoir. According to one embodiment, the reservoir has the form of a bubble. The fact that the bubble does not break into several drops is linked to the surface tension, to the interfacial tension and to the viscosity of the composition. These three physico-chemical properties of the composition may be considered as close to the one of the oil phase used in the composition. As a matter of example, medium chain triglycerids (MCT) present a surface tension of 30 mN/m, an interfacial tension of 45 mN/m and viscosity ranging from 27 to 33 mPa.s at 20 °C and this combination of physico-chemical properties avoids MCT to break into several oil drops.

According to one embodiment, the viscosity of the composition is ranging from 5 to 10 000 mPa.s at 20°C depending on the amount of water emulsified in the oil, preferably ranging from 25 to 5000 mPa.s at 20°C, preferably ranging from 24 to 1000 mPa.s at 20°C, preferably ranging from 25 to 500 mPa.s at 20°C. According to one embodiment, the viscosity of the composition is ranging from 5 to 100 mPa.s at 20°C, preferably from 5 to 50 mPa.s at 20°C, more preferably from 5 to 20 mPa.s at 20°C. According to another embodiment, the viscosity of the composition is ranging from 100 to 10 000 mPa.s at 20°C, preferably from 500 to 10 000 mPa.s at 20°C, more preferably from 5000 to 10 000 mPa.s at 20°C. According to the invention, the viscosity is measured using a Kinexus Pro from Malvern U.K. at 20°C.

According to one embodiment, the surface tension of the composition is ranging from 0 to 30 mN/m, preferably ranging from 5 to 20 mN/m, more preferably ranging from 10 to 15 mN/m.

According to one embodiment, the interfacial tension of the composition is ranging from 0 to 45 mN/m, preferably ranging from 5 to 30 mN/m, more preferably ranging from 10 to 20 mN/m.

The water-in-oil type emulsion of the invention is efficient for sustained release administration of a therapeutic agent. According to one embodiment, the therapeutic agent is released for a period of time ranging from 2 weeks to 12 months, preferably from 1 month to 6 months.

The sustained release effect is provided by the migration of water droplets dispersed in the continuous oil phase to the surface of the oil reservoir formed by the emulsion when injected in the eye. In one embodiment of the invention, the sustained release kinetic can be adapted to the exact need of the patient.

In a first embodiment of the invention, said sustained release kinetic may depend on the viscosity of the oil phase. Indeed, the more viscous the oil phase is, the more extended the period of release may be, as evidenced by the Stokes law:

$$v_s = \frac{2}{9} \frac{(\rho_p - \rho_f)}{\mu} g\, R^2$$

where:

- $v_s$ is the particles' settling velocity (m/s) (vertically downwards if pp > pf, upwards if pp < $\rho$f),
- g is the gravitational acceleration (m/s$^2$),
- pp is the mass density of the particles (kg/m$^3$), and
- pf is the mass density of the continuous phase (kg/m3).
- R the radius of the particle.
- $\mu$ is the viscosity of the continuous phase

The Stokes law states that the speed of movement of a particle (water droplet), in a continuous phase (oil phase), is inversely proportional to the viscosity ($\mu$) of the continuous phase. Therefore, the higher the viscosity of the oil phase of the emulsion is, the slower the water droplets will move to the surface of the oil reservoir to release the therapeutic agent. With viscous oil such as long chain triglycerides, the release of the therapeutic agent may be extended up to one year. According to an embodiment, the viscosity of the oil phase ranges from 1 to 10000 mPa.s at 20°C, preferably from 10 to 5000 mPa.s at 20°C, even more preferably from 25 to 1000 mPa.s at 20°C.

In a second embodiment of the invention, said sustained release kinetic may depend on the size of the water droplets dispersed in the oil phase. Indeed, a bigger water droplet will move faster than a smaller as described by the Stokes law. Therefore, the smaller the droplets are, the longer their migration to the surface of the injected reservoir may be, and then the more extended the period of release of the therapeutic agent may be. For example, for comparable compositions of the invention in terms of ingredients, an emulsion with a droplet size of more than 1 $\mu$m may release the therapeutic agent in about 1 week to 2 months, whereas the release may be increased to more than 2 months when the droplet size is below 500 nm. According to an embodiment, the size of the water droplets in the emulsion of the invention ranges from 1 to 2000 nm, preferably from 10 to 1000 nm, more preferably from 20 to 600 nm.

In a third embodiment of the invention, said sustained release kinetic may be conditioned by the volume of the injected water-in-oil type emulsion. The bigger the emulsion reservoir is, the more extended the period of release may be. Indeed, the bigger the emulsion reservoir is, the longer the road of the water droplet to reach the surface of the reservoir is. Preferably, a volume of the composition of the invention ranging from 5 to 250 $\mu$L, preferably from 10 to 100 $\mu$L, more preferably about 50 $\mu$L is injected.

In a fourth embodiment of the invention, the viscosity of the aqueous phase is increased in order to enhance the sustained release. In a particular embodiment of the invention, said viscosity is increased by addition of a viscosity modifying agent selected from the group comprising sodium hyaluronate, carbopol gels, hydroxyethyl cellulose, dextran, carboxymethyl cellulose, PEG, polyvinyl alcohol, collagen. In a preferred embodiment of the invention, said hydrogel is made of cellulose, hyaluronic acid, and/or collagen.

In a particular embodiment of the present invention, the water-in-oil emulsion is deprived of organogelling agent such as amino acid derivatives, especially fatty acid ester derivatives of amino acids, more specifically alanine ester derivatives. In this particular embodiment, organogelling agents refer to molecules which have the capacity to self-assemble spontaneously via bonds of low energy to form a matrix that immobilizes hydrophobic organic liquid. In a particular embodiment, the water-in-oil emulsion of the present invention is not a phase transition system.

In a fifth embodiment of the invention, the means for sustaineously releasing the therapeutic agents as described in the first to four embodiments hereabove, may be combined one to each other or all together in order to modulate the sustain release effect.

According to an embodiment of the invention, the aqueous phase of the emulsion further comprises a pH modifying agent or a pH buffering agent. In a preferred embodiment, said pH buffering agent is selected from the group comprising phosphate, citrate, tris, histidine or acetate buffers. In a preferred embodiment, said pH buffering agent is a phosphate buffer. In one embodiment of the invention, the amount of said agent for modifying the pH of the aqueous phase ranges from 0.05 to 10% in weight to the total weight of the aqueous phase, preferably from 0.01 to 5% w/w, more preferably from 0.1 to 1% w/w.

According to an embodiment of the invention, the aqueous phase of the emulsion further comprises an agent for modifying the osmolality of the aqueous phase of the emulsion. In a first embodiment, said agent for modifying the osmolality is selected from the group comprising NaCl, KCl and CaCl$_2$. In a second embodiment, the modification of the osmolality of the composition results from the addition of a compound selected from the group comprising neutral compounds such as glycerol, mannitol, alpha-trehalose or propylene glycol. In a preferred embodiment, the modification of the osmolality of the composition results from the addition of 0.5-2%, preferably 0.9% w/w of NaCl, 0.5-10%, preferably 3-5% w/w of alpha-trehalose or mannitol or propylene glycol in weight to the weight of the total emulsion.

In a particular embodiment, water-in-oil emulsions of the present invention are not double emulsions (i.e. water-in-oil-in-water or oil-in-water-in-oil emulsions).

According to an embodiment, the composition is intraocularly injected. Preferably, the composition is intravitreally injected.

The water-in-oil type emulsion according to the invention is bioresorbable. In one embodiment of the invention, the oily reservoir is resorbed in a period of time ranging from 1 to 24 months after injection, preferably from 6 to 18 months after injection, more preferably about 12 months after injection.

The water-in-oil type emulsion according to the invention is for use in treating diseases or conditions of the eye. In one embodiment of the invention, said diseases or conditions of the eye are selected from the group comprising glaucoma, anterior uveitis retinal oxidation, age related macular degeneration, posterior uveitis, diabetic macular edema and central vein occlusion.

The present invention also relates to a pharmaceutical composition according to the water-in-oil type emulsion of the invention. In one embodiment of the invention, the pharmaceutical composition further comprises at least one pharmaceutically acceptable excipient.

The present invention also relates to a medicament according to the water-in-oil type emulsion of the invention.

The present invention also relates to a device for administering the water-in-oil type emulsion, the pharmaceutical composition or the medicament according to the invention. Preferably, said device is a prefilled syringe comprising 20 $\mu$L to 350 $\mu$L of the composition of the invention. In one embodiment of the invention, said device contains the pharmaceutical composition or the medicament according to the invention.

Also, the present invention relates to a composition for use in the treatment by intraocular injection of a condition or disease of the eye, comprising administering intraocularly a therapeutic amount of the composition or of the medicament of the invention. Preferably, the method of the invention comprises the injection, preferably in the vitreous chamber, of a volume ranging from 5 to 250 $\mu$L, preferably from 10 to 100 $\mu$L, more preferably of about 50 $\mu$L. In a preferred embodiment, said composition or medicament is injected less than once a week, preferably less than once a month, more preferably less than once in six months. According to an embodiment, the injected composition forms *in situ* a reservoir within which the aqueous phase migrates towards the surface of the reservoir, letting the therapeutic agent be sustaineously released to the vitreous chamber or the targeted tissue. According to one embodiment, the reservoir has the form of a bubble. According to another embodiment, the reservoir has the form of a spread bubble. According to another embodiment, the reservoir has the form of a layer, floating over the vitreous liquid.

The water-in-oil emulsion of the invention may be manufactured either through a conventional process or through a process called membrane emulsification.

In the conventional process, the oily phase components are successively weighed in the same beaker and then magnetically stirred under a slight heating (30-50°C, preferably 40°C) until a slightly viscous phase is obtained. Aqueous phase components are successively weighed in the same beaker and then magnetically stirred under a slight heating (30-50°C, preferably 40°C) until a transparent, limpid and fluid phase is obtained. Both phases are heated (to 50-80°C, preferably 65°C). The emulsion droplet size may be decreased by high sheer mixing a 5 minutes high shear mixing with a POLYTRON PT 6100. The emulsion may be homogenized in a microfluidizer (C5, Avestin).

An alternative manufacturing process is membrane emulsification: the emulsions of the invention may also be manufactured by membrane emulsification as described by Serguei (Serguei R. Kosvintsev, Gilda Gasparini, Richard G. Holdich, Membrane emulsification: droplet size and uniformity in the absence of surface shear, Journal of Membrane Science, Volume 313, Issues 1-2, 10 April 2008, Pages 182-189). In this alternative process, the oily phase components are successively weighed in the same beaker and then magnetically stirred under a slight heating (30-50°C, preferably 40°C) until a slightly viscous phase is obtained. Aqueous phase components are successively weighed in the same beaker and then magnetically stirred under a slight heating (30-50°C, preferably 40°C) until a transparent, limpid and fluid phase is obtained. Both phases are heated (to 50-80°C, preferably 65°C). Aqueous phase is forced through a membrane with 1 $\mu$m pores. The water droplets are collected by a continuous flux of the oily phase.

BRIEF DESCRIPTION OF THE DRAWINGS

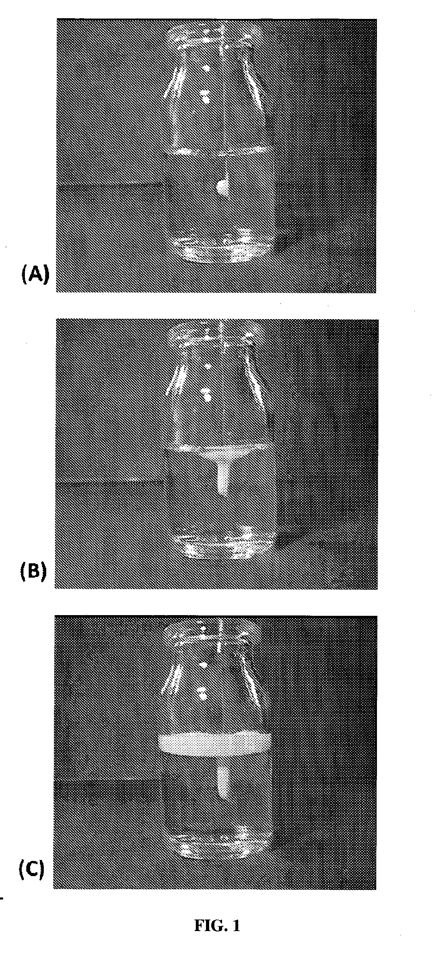

Figure 1 represents photographs showing the injection of 60 $\mu$L of the composition of example 1 in a glass of water and behavior of the composition 16 seconds (FIG. 1A), 24 seconds (FIG. 1B) and 1 minute (FIG. 1C) after injection.

EXAMPLES

The present invention is further illustrated by the following examples.

Example 1: Composition

This composition is a water-in-oil emulsion as described in the specification, obtained by either one of the manufacturing processes described below using the following ingredients in indicated amounts:

| Ingredients | Concentration % w/w |
| --- | --- |
| Ranibizumab | 0.1% |
| Water for injection | 4% |
| Dihydrated alpha, alpha-trehalose | 3% |
| Monohydrated histidine chlorhydrate histidine | 0.05% |
| Sorbitan stearate | 2% |
| Medium chain triglyceride | Qs 100% |

Manufacturing process:

The oily phase components were successively weighed in the same beaker and then magnetically stirred under a slight heating until a slightly viscous phase is obtained. Aqueous phase components were successively weighed in the same beaker and then magnetically stirred under a slight heating (40°C) until a transparent, limpid and fluid phase is obtained. Both phases were heated to 65°C. The coarse emulsion is formed by rapid addition of the aqueous phase in the oily phase. The emulsion is white and slightly transparent. The emulsion droplet size is decreased by applying a 5 minute high shear mixing with a POLYTRON PT 6100. The emulsion became milky. The emulsion temperature was cooled down to 20°C.

The final emulsion was obtained by homogenization in a microfluidizer (C5, Avestin) using continuous cycles for 5 min at a pressure of 10 000 psi. The emulsion was milky and very fluid. The emulsion temperature was decreased to 25°C.

Characterization:

Emulsion was conditioned in glass vials with nitrogen bubbling and then sterilized in an autoclave 20 minutes at 121°C. The mean particle size of the emulsions droplets was determined by quasi-elastic light scattering after dilution in water using a High Performance Particle Sizer (Malvern Instruments, UK) . The electrophoretic mobility was measured at 25°C in a Malvern Zetasizer 2000 (Malvern Instruments, UK) following a 1:200 dilution in double distilled water as detailed above and converted into zeta potential through the Smoluchowski equation. The viscosity is measured using a Kinexus Pro from Malvern U.K. at 20°C. The density was measured by filling a calibrated volumetric flask with the emulsion and weighed on a balance. Volume/mass ratio is then calculated.

Specifications of the composition of example 1:

| Size of the water droplets | Density | In vitro release time of ranibizumab |
|---|---|---|
| 500 nm | 0.94 g/cm$^3$ | 2 months |

In vitro release test is performed by incubating at 37°C, 20µL of the composition in 4 mL of water. Quantification of active ingredient release in water is done by HPLC. At 2 months the entire quantity of ranibizumab was released in the water.

An in vitro test of injection was performed by injecting 60 µL of composition of example 1 in water. As shown in Figure 1, the composition reaches the surface as soon as injected in the aqueous media. This result is linked to the density of the composition that is lower than water's.

Example 2: composition comprising sodium pegaptanib

| Ingredients | Concentration |
|---|---|
| Sodium pegaptanib | 0.8% |
| Water for injection | 5% |
| Glycerol monostearate | 0.5% |
| Sorbitan monopalmitate | 1% |
| Medium chain triglyceride | Qs 100% |
| Dexamethasone palmitate | 1.2% |

Specifications of the composition of example 2:

| Size of the water droplets | Density | In vitro release time of pegaptanib |
|---|---|---|
| 200 nm | 0.95 g/cm$^3$ | 4 months |

As in example 1, in vitro release test is performed by incubating at 37°C 20µL of the composition in 4 ml of water. Quantification is done by HPLC.

Compared to example 1, and in accordance with the Stokes law, with a decrease of the water droplet size the release time has doubled, confirming that size of the dispersed droplets is a key factor in the release rate of the hydrophilic active ingredient.

**Claims**

1.  A composition for use in the treatment by intraocular route of diseases or conditions of the eye, wherein the composition is a water-in-oil type emulsion comprising an oil phase, a lipophilic surfactant dissolved in the oil phase, an aqueous phase dispersed in the oil phase, a hydrophilic therapeutic agent dissolved in the aqueous dispersed phase, and wherein the composition has a density lower than 1, preferably ranging from 0.91 to 0.97 g/cm$^3$, wherein the composition has a viscosity ranging from 25 to 10 000 mPa.s at 20 °C, wherein the size of the droplets of water ranges from 20 nm to 600 nm, and wherein the use by intraocular route is intraocular injection.

2.  The composition for use of claim **1**, wherein the oil phase is selected from the group comprising triglycerides such as, for example, medium chain or long chain triglycerides, monoglycerides, diglycerides, vegetable oils or mineral oils.

3.  The composition for use according to anyone of claim **1** or claim **2**, wherein the lipophilic surfactant is selected from the group comprising sorbitan ester such as, for example, sorbitan stearate, sorbitan laurate and sorbitan monopalmitate; bentonite; glycerol monostearate; propylene glycol monolaurate and mixtures thereof.

4.  The composition for use according to anyone of claims **1** to **3**, wherein the aqueous phase is present in an amount ranging from 0.1 to less than 50% in weight to the total weight of the composition, preferably from 0.5 to 15% w/w, more preferably from 2 to 10% w/w.

5.  The composition for use according to anyone of claims **1** to **4**, wherein said hydrophilic therapeutic agent is selected from the group comprising monoclonal antibodies (full or fragment Fab), such as for example ranibizumab, bevacizumab trastuzumab, cituximab or rituximab; anti-angiogenic molecules, such as for example pegaptanib; a ROCK (Rho-kinases) inhibitor, such as for example fasudil; proteins such as anti-CD 160 S-HLA-G; WNT3A protein which activates WNT (Wingless - Integration site) for survival of photoreceptor cells; growth factors such as epithelium growth factors (EGF), anti-EGF or TGF (Transforming growth factor); siRNA such as siRNA anti-arginase, miRNA; oligonucleotides such as antisens DNA or antisens RNA; iron chelating molecules such as deferiprone and salicylaldehyde isonicotinoyl hydrazone; anti-inflammatory molecules such as epigallocatechin gallate; or antibiotics for back of the eye infection such as linezolide, clavulamic acid, macrolides, anti-inflammatory molecules preferably selected from the group comprising, cortico-steroids such as dexamethasone and its hydrophilic derivatives, or mixtures thereof.

6.  The composition for use according to anyone of claims **1** to **5**, further comprising a lipophilic therapeutic agent dissolved in the oil phase, said lipophilic therapeutic agent being selected from the group comprising cyclosporine A lutein, alpha-tocopherol and dexamethasone palmitate.

7.  The composition for use according to anyone of claims **1** to **6**, further comprising viscosity modifying agents, such as, for example an hydrogel of sodium hyaluronate, carbopol gels, hydroxyethyl cellulose, dextran, carboxymethyl cellulose, PEG, polyvinyl alcohol, collagen, and/or pH buffering agents, such as, for example, phosphate, citrate, tris, histidine or acetate buffer, and/or osmolality modifying agents, such as, for example NaCl, KC1, CaCl$_2$, glycerol, mannitol, alpha-trehalose or propylene glycol.

8.  The composition for use according to anyone of claims **1** to **7**, wherein the use by intraocular route is intravitreal injection.

9.  The composition for use according to anyone of claims **1** to **8**, wherein said diseases or conditions of the eye to be treated are selected from the group comprising glaucoma, anterior uveitis retinal oxidation, age related macular degeneration, posterior uveitis, diabetic macular edema and central vein occlusion.

10. The composition for use in the treatment by intraocular route of diseases or conditions of the eye according to anyone of claims **1** to **9**, which is a pharmaceutical composition comprising one or more pharmaceutically acceptable excipients.

11. The composition for use in the treatment by intraocular route of diseases or conditions of the eye according to anyone of claims **1** to **9**, which is a medicament.

12. The composition for use in the treatment by intraocular route of diseases or conditions of the eye according to anyone of claims **1** to **9**, wherein a volume ranging from 5 to 250 microliters of the composition is administered by intraocular route, wherein a therapeutic amount of a hydrophilic therapeutic agent is dissolved in the aqueous dispersed phase.

13. The composition for use in the treatment by intraocular route of diseases or conditions of the eye according to anyone of claims **1** to **9**, which is comprised in a device.

14. The composition for use in the treatment by intraocular route of diseases or conditions of the eye according to anyone of claims **1** to **9**, which is comprised in a device comprising a volume of 20 to 350 microliters of the composition.

**Patentansprüche**

1. Zusammensetzung zur Verwendung bei der Behandlung von Erkrankungen oder Zuständen des Auges auf intraokularem Weg,
   wobei die Zusammensetzung eine Emulsion vom Typ Wasser-in-Öl ist, umfassend eine Ölphase, ein lipophiles Tensid, das in der Ölphase gelöst ist, eine wässrige Phase, die in der Ölphase dispergiert ist, ein hydrophiles therapeutisches Mittel, das in der wässrigen dispergierten Phase gelöst ist, und
   wobei die Zusammensetzung eine Dichte von weniger als 1 aufweist, vorzugsweise im Bereich von 0,91 bis 0,97 g/cm$^3$,
   wobei die Zusammensetzung eine Viskosität aufweist, die im Bereich von 25 bis 10000 mPa.s bei 20 °C liegt,
   wobei die Größe der Wassertröpfchen von 20 nm bis 600 mn reicht,
   und wobei die Verwendung auf intraokularem Weg eine intraokulare Injektion ist.

2. Zusammensetzung zur Verwendung nach Anspruch **1**, wobei die Ölphase ausgewählt ist aus der Gruppe, bestehend aus Triglyceriden wie z.B. mittelkettigen oder langkettigen Triglyceriden, Monoglyceriden, Diglyceriden, pflanzlichen Ölen oder Mineralölen.

3. Zusammensetzung zur Verwendung nach einem von Anspruch **1** oder Anspruch **2**, wobei das lipophile Tensid ausgewählt ist aus der Gruppe, bestehend aus Sorbitanester wie z.B. Sorbitanstearat, Sorbitanlaurat und Sorbitanmonopalmitat; Bentonit, Glycerolmonostearat; Propylenglykolmonolaurat und Mischungen davon.

4. Zusammensetzung zur Verwendung nach einem der Ansprüche **1** bis **3**, wobei die wässrige Phase in einer Menge vorhanden ist, die von 0,1 bis weniger als 50 Gew.% zum Gesamtgewicht der Zusammensetzung reicht, vorzugsweise von 0,5 bis 15 Gew.%, insbesondere von 2 bis 10 % Gew.%.

5. Zusammensetzung zur Verwendung nach einem der Ansprüche **1** bis **4**, wobei das hydrophile therapeutische Mittel ausgewählt ist aus der Gruppe bestehend aus monoklonalen Antikörpern (Voll- oder Fragment-Fab) wie z.B. Ranibizumab; Bevacizumab, Trastuzumab, Cetuximab oder Rituximab; anti-angiogenen Molekülen wie z.B. Pegaptanib; einem ROCK (Rho-Kinase)-Hemmer, wie z.B. Fasudil; Proteinen wie z.B. Anti-CD160 S-HLA-G; WNT3A-Protein, das WNT (Wingless-Integrationsstelle) zum Überleben von Photorezeptorzellen aktiviert; Wachstumsfaktoren wie z.B. Epithelwachstumsfaktoren (EGF), Anti-EGF oder WIT (Transformationswachstumsfaktor); siRNA wie z.B. siRNA Anti-Arginase, miRNA; Oligonukleotiden wie z.B. Antisens-DNA oder Antisens-RNA; Eisenchelatierungsmolekülen wie z.B. Deferipron und Salicylaldehydisonicotinoylhydrazon; entzündungshemmenden Molekülen wie z.B. Epigallocatechingallat; oder Antibiotika für Infektionen der hinteren Augenabschnitte wie z.B. Linezolid, Clavulansäure, Makrolide, entzündungshemmenden Molekülen, vorzugsweise ausgewählt aus der Gruppe bestehend aus Corticosteroiden wie z.B. Dexamethason und seinen hydrophilen Derivaten oder Mischungen daraus.

6. Zusammensetzung zur Verwendung nach einem der Ansprüche **1** bis **5**, weiter umfassend ein lipophiles therapeutisches Mittel, das in der Ölphase gelöst ist, wobei das lipophile therapeutische Mittel ausgewählt ist aus der Gruppe bestehend aus Cyclosporin A Lutein, Alpha-Tocopherol und Dexamethasonpalmitat.

7. Zusammensetzung zur Verwendung nach einem der Ansprüche **1** bis **6**, weiter umfassend viskositätsmodifizierende Mittel wie z.B. ein Natriumhyaluronathydrogel, Carbopolgels, Hydroxyethylcellulose, Dextran, Carboxymethylcellulose, PEG, Polyvinylalkohol, Kollagen und/oder pH-Puffermittel wie z.B. Phosphat, Citrat, Tris, Histidin oder Acetatpuffer und/oder Mittel zur Modifizierung der Osmolalität wie z.B. NaCl, KCl, CaCl$_2$, Glycerol, Mannitol, alpha-Trehalose oder Propylenglykol.

**8.** Zusammensetzung zur Verwendung nach einem der Ansprüche **1** bis **7**, wobei die Verwendung auf intraokularem Weg eine intraokulare Injektion ist.

**9.** Zusammensetzung zur Verwendung nach einem der Ansprüche **1** bis **8,** wobei die Erkrankungen oder Zustände des Auges, die behandelt werden sollen, ausgewählt sind aus der Gruppe, bestehend aus Glaucoma, Netzhautoxidation der anterioren uveitis, altersbedingter Makuladegeneration, posteriorer Uveitis, diabetischem Makulaödem und zentralem Venenverschluss.

**10.** Zusammensetzung zur Verwendung bei der Behandlung von Erkrankungen oder Zuständen des Auges auf intraokularem Weg nach einem der Ansprüche **1** bis **9,** wobei es sich um eine pharmazeutische Zusammensetzung handelt, umfassend einen oder mehrere pharmazeutisch annehmbare Trägerstoffe.

**11.** Zusammensetzung zur Verwendung bei der Behandlung von Erkrankungen oder Zuständen des Auges auf intraokularem Weg nach einem der Ansprüche **1** bis **9,** wobei es sich um ein Arzneimittel handelt.

**12.** Zusammensetzung zur Verwendung bei der Behandlung von Erkrankungen oder Zuständen des Auges auf intraokularem Weg nach einem der Ansprüche **1** bis **9,** wobei ein Volumen im Bereich von 5 bis 250 Mikroliter der Zusammensetzung auf intraokularem Weg verabreicht wird, wobei eine therapeutische Menge eines hydrophilen therapeutischen Mittels in der wässrigen dispergierten Phase gelöst ist.

**13.** Zusammensetzung zur Verwendung bei der Behandlung von Erkrankungen oder Zuständen des Auges auf intraokularem Weg nach einem der Ansprüche **1** bis **9,** die in einer Vorrichtung enthalten ist.

**14.** Zusammensetzung zur Verwendung bei der Behandlung von Erkrankungen oder Zuständen des Auges auf intraokularem Weg nach einem der Ansprüche **1** bis **9,** die in einer Vorrichtung enthalten ist, umfassend ein Volumen von 20 bis 350 Mikroliter der Zusammensetzung.

**Revendications**

**1.** Composition pour son utilisation dans le traitement par voie intraoculaire de maladies ou de conditions de l'oeil, dans laquelle la composition est une émulsion de type eau-dans-huile comprenant une phase huileuse, un surfactant lipophile dissous dans la phase huileuse, une phase aqueuse dispersée dans la phase huileuse, un agent thérapeutique hydrophile dissous dans la phase aqueuse dispersée, et
dans laquelle la composition a une densité inférieure à 1, de préférence de 0.91 à 0.97 g/cm$^3$,
dans laquelle la composition a une viscosité allant de 25 à 10 000 mPa.s à 20°C, dans laquelle la taille des gouttes d'eau est de 20 nm à 600 nm, et
dans laquelle l'utilisation par voie intraoculaire est l'injection intraoculaire.

**2.** La composition pour son utilisation selon la revendication **1,** dans laquelle la phase huileuse est sélectionnée dans le groupe comprenant les triglycérides tels que par exemple les triglycérides à chaîne moyenne ou longue, les monoglycérides, les diglycérides, les huiles végétales ou les huiles minérales.

**3.** La composition pour son utilisation selon la revendication **1** ou la revendication **2,** dans laquelle le surfactant lipophile est sélectionné dans le groupe comprenant les esters de sorbitan tels que par exemple le stéarate de sorbitan, le laurate de sorbitan et le monopalmitate de sorbitan ; la bentonite, le monostéarate de glycérol ; le monolaurate de propylène glycol ou leurs mélanges.

**4.** La composition pour son utilisation selon l'une quelconque des revendications **1** à 3, dans laquelle la phase aqueuse est présente dans une quantité allant de 0,1 à moins de 50% en poids par rapport au poids total de la composition, de préférence de 0,5 à 15% p/p, plus préférentiellement de 2 à 10% p/p.

**5.** La composition pour son utilisation selon l'une quelconque des revendications **1** à **4,** dans laquelle ledit agent thérapeutique hydrophile est sélectionné dans le groupe comprenant les anticorps monoclonaux (entiers or fragments Fab), tels que par exemple le ranibizumab, bevacizumab, trastuzumab, cituximab ou rituximab; les molécules anti-angiogéniques telles que par exemple le pegaptanib; un inhibiteur de ROCK (Rho-kinases), tel que par exemple le fasudil; les protéines telles que anti-CD160 S-HLA-G; la protéine WNT3A qui active le WNT (site d'intégration Wingless) pour la survie des cellules photoréceptrices; les facteurs de croissance tels que les facteurs de croissance de l'épithélium (EGF), anti-EGF ou TGF (facteur de croissance transformant); les siARN tel que le siARN anti-arginase, les miARN; les oligonucléotides tels que l'ADN antisens ou l'ARN antisens; les molécules chélatant le fer telles que la défériprone et l'hydrazone d'isonicotinoyle salicylaldéhyde; les molécules anti-inflammatoires telles que le gallate d'épigallocatéchine; ou les antibiotiques pour les infections de l'arrière de l'oeil tels que le linézolide, l'acide clavulamique, les macrolides, les molécules anti-inflammatoires sélectionnées de préférence dans le groupe comprenant les corticostéroïdes tels que la dexaméthasone et ses dérivés hydrophiles, ou leurs mélanges.

6. La composition pour son utilisation selon l'une quelconque des revendications **1** à **5,** comprenant en outre un agent thérapeutique lipophile dissous dans la phase huileuse, ledit agent thérapeutique lipophile étant sélectionné dans le groupe comprenant la cyclosporine, la lutéine, l'alpha-tocophérol et la dexaméthasone palmitate.

7. La composition pour son utilisation selon l'une quelconque des revendications **1** à **6,** comprenant en outre des agents de modification de la viscosité, tels que par exemple un hydrogel de hyaluronate de sodium, des gels carbopol, hydroxyéthyl cellulose, dextran, carboxyméthyl cellulose, PEG, polyvinyl alcool, collagène, et/ou des agents tampon de pH, tels que par exemple un tampon phosphate, citrate, tris, histidine ou acétate, et/ou des agents de modification de l'osmolalité, tels que par exemple NaCl, KCl, $CaCl_2$, glycérol, mannitol, alpha-tréhalose ou propylène glycol.

8. La composition pour son utilisation selon l'une quelconque des revendications **1** à **7,** dans laquelle l'utilisation par voie intraoculaire est l'injection intravitréenne.

9. La composition pour son utilisation selon l'une quelconque des revendications **1** à **8,** dans laquelle lesdites maladies ou conditions de l'oeil à traiter sont sélectionnées dans le groupe comprenant le glaucome, l'uvéite antérieure, l'oxydation de la rétine, la dégénérescence maculaire liée à l'âge, l'uvéite postérieure, l'oedème maculaire diabétique et l'occlusion de la veine centrale.

10. La composition pour son utilisation dans le traitement par voie intraoculaire de maladies ou de conditions de l'oeil selon l'une quelconque des revendications **1** à **9,** qui est une composition pharmaceutique comprenant un ou plus excipients pharmaceutiquement acceptables.

11. La composition pour son utilisation dans le traitement par voie intraoculaire de maladies ou de conditions de l'oeil selon l'une quelconque des revendications **1** à **9,** qui est un médicament.

12. La composition pour son utilisation dans le traitement par voie intraoculaire de maladies ou de conditions de l'oeil selon l'une quelconque des revendications **1** à **9,** dans laquelle un volume de 5 à 250 microlitres de la composition est injectée par voie intraoculaire, dans laquelle une quantité thérapeutique de l'agent thérapeutique hydrophile est dissous dans la phase aqueuse dispersée.

13. La composition pour son utilisation dans le traitement par voie intraoculaire de maladies ou de conditions de l'oeil selon l'une quelconque des revendications **1** à **9,** qui est comprise dans un dispositif.

14. La composition pour son utilisation dans le traitement par injection intraoculaire de maladies ou de conditions de l'oeil selon l'une quelconque des revendications **1** à **9,** qui est comprise dans un dispositif comprenant un volume de 20 à 350 microlitres de la composition.

FIG. 1

**EP 2 611 414 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009046198 A
- EP 2187980 A
- WO 0189479 A

**Non-patent literature cited in the description**

- **TAMILVANAN.** *Progress in Lipid Research,* 2004, vol. 43, 489-533
- **SERGUEI R. KOSVINTSEV ; GILDA GASPARINI ; RICHARD G. HOLDICH.** Membrane emulsification: droplet size and uniformity in the absence of surface shear. *Journal of Membrane Science,* 10 April 2008, vol. 313 (1-2), 182-189